# FASCICULE DE BREVET EUROPEEN

(11) **EP 1 886 141 B1**
(45) Date de publication et mention de la délivrance du brevet: **01.06.2011**
(21) Numéro de dépôt: 06764700.8
(22) Date de dépôt: 31.05.2006
(51) Int. Cl.: G01N 33/532

(54) **PROCÉDÉ DE MARQUAGE OU DE TRAITEMENT D'UN ÉCHANTILLON BIOLOGIQUE CONTENANT DES ACIDES NUCLÉIQUES**
VERFAHREN ZUR MARKIERUNG ODER BEHANDLUNG EINER BIOLOGISCHEN PROBE, DIE NUKLEINSÄUREN ENTHÄLT
METHOD FOR LABELLING OR TREATING A BIOLOGICAL SAMPLE CONTAINING NUCLEIC ACIDS

(30) Priorité: 01.06.2005 FR 0551452
(43) Date de publication de la demande: 13.02.2008
(73) Titulaire: bioMérieux S.A., 69280 Marcy l'Etoile (FR)
(72) Inventeur: BERNAL-MENDEZ, Eloy, F-38070 Saint Quentin Fallavier (FR); LAAYOUN, Ali, F-38690 Colombe (FR); MENOU, Lionel, F-69230 Saint Genis Laval (FR)
(86) Numéro de dépôt international: PCT/FR2006/001228
(87) Numéro de publication internationale: WO 2006/129010

(56) Documents cités:
- WO-A1-02/090319
- FR-A1- 2 868 071
- US-A- 5 244 816
- US-B1- 6 414 136
- LAAYOUN A ET AL: "Aryldiazomethanes for Universal Labeling of Nucleic Acids and Analysis on DNA Chips" BIOCONJUGATE CHEMISTRY, ACS, WASHINGTON, DC, US, vol. 14, 2003, pages 1298-1306, XP002308387 ISSN: 1043-1802

## Description

La présente invention concerne un nouveau procédé de purification d'un échantillon biologique contenant des acides nucléiques d'intérêt, acides ribonucléiques (ARN) ou acides désoxyribonucléiques (ADN) de synthèse ou naturel, qui ont été marqués, telle que défini dans les revendications.

Par "ARN ou ADN de synthèse", il faut comprendre ARN ou ADN obtenu par une technique mise au point par l'homme, par exemple une technique d'amplification (PCR suivie éventuellement d'une transcription) ou d'amplification transcriptionnelle (TMA ou NASBA). Par "ARN ou ADN naturel", il faut comprendre ARN ou ADN obtenu par extraction d'une cellule, par exemple ARN messager, ribosomal, de transfert ou ADN génomique.

L'état de la technique montre que de nombreuses méthodes existent pour marquer de tels nucléotides, oligonucléotides ou acides nucléiques. Les oligonucléotides et les acides nucléiques seront tous ultérieurement désignés par le terme polynucléotides. Le marquage peut s'effectuer soit lors de la synthèse, soit par incorporation d'au moins un nucléotide marqué.

Une première méthode consiste à fixer le marqueur sur la base, que celle-ci soit naturelle ou modifiée. Une deuxième méthode propose de fixer le marqueur sur le sucre, la encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer un marquage d'un nucléotide, ou d'un analogue de nucléotide ou d'un polynucléotide, est enclin à effectuer cette fixation sur la base ou sur le sucre qui lui offrent plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16094, DE-A-3.910.151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem Soc., 82, 1778-1785, 1960). De même dans la revue de O'Donnel et Mc Laughlin (« Reporter groups for the analysis of nucleic acid structure », p 216-243, dans « Bioorganic Chemistry : Nucleic Acids », Ed Hecht S.M., Oxford University Press, 1996) portant sur les méthodes d'introduction de sondes dans les fragments d'oligonucléotides, l'alkylation efficace du phosphodiester internucléotidique est considérée comme étant impossible.

La Demanderesse a d'ores et déjà développé une technique de marquage basée sur de nouveaux réactifs qui soient efficaces du point de vue du rendement de marquage, qui soient spécifiques au niveau de la position de marquage et en particulier qui n'affectent pas les propriétés d'hybridation des bases impliquées dans la formation de la double hélice, par l'intermédiaire des liaisons hydrogènes, qui soient utilisables à la fois pour l'ADN et l'ARN, et enfin qui permettent de marquer indifféremment des polynucléotides naturels ou préparés par amplification enzymatique.

Ainsi dans la demande de brevet WO-A-02/090319 sont décrits de nouveaux marqueurs qui répondent aux conditions précitées et qui utilisent la fonction diazométhyle comme fonction réactive pour le marquage. La fonction diazométhyle (de formule -C(N₂)-) a déjà été utilisée pour l'alkylation des groupements phosphates, mais un certain nombre de problèmes se posent. D'une part, les dérivés diazo en général sont instables eux-mêmes, ce qui pose des problèmes pour l'utilisation des ces réactifs de marquage dans un kit de marquage, et d'autre part, le produit de couplage est instable ce qui est rédhibitoire si le produit marqué a pour fonction de mettre en évidence la présence d'une molécule cible biologique dans un échantillon quelconque. Enfin les dérivés portant la fonction diazométhyle sont insolubles dans l'eau, ce qui conduit à utiliser des conditions biphasiques pour le couplage avec des molécules biologiques, qui ne sont solubles et stables que dans l'eau ou des tampons aqueux, mais ces conditions ralentissent la vitesse de réaction et donc nuisent à l'efficacité du couplage. Les nouveaux réactifs de marquage de cette invention, décrite dans la demande WO-A-02/090319, résolvent aussi ces problèmes techniques. Selon un mode de réalisation, le réactif de marquage stable à la température est de formule : 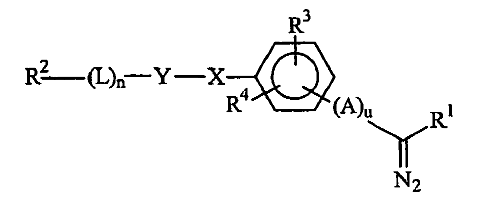 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO_{2,} Cl, Br, F, I, R² -(L)ₙ-Y-X-, OR, SR, NR₂, R, NHCOR, CONHR, COOR avec R = alkyle ou aryle,
- A est un bras de liaison comportant au moins une double liaison covalente permettant la conjugaison de la fonction diazo avec le cycle aromatique et u est un nombre entier compris entre 0 et 2, préférentiellement de 0 ou 1, et
- -Y-X- représente -CONH-, -NHCO-, -CH₂O-, -CH₂S-.

Dans une nouvelle demande de brevet déposée par la Demanderesse sous le numéro FR04/50600 en date du 26 mars 2004, et intitulée : "Réactifs de marquage, procédés de synthèse de tels réactifs et procédés de détection de molécules biologiques", ces molécules basées sur la fonction diazo ont encore été améliorées, sont toujours stables à la température et de formule : 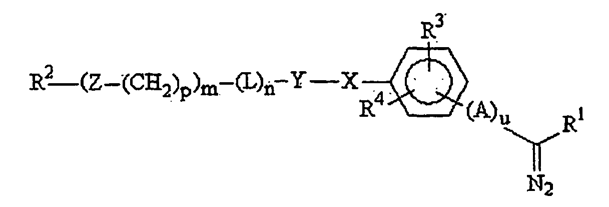 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², -CO-NH- (CH₂)₃- (O-CH₂-CH₂)₄-CH₂-NH-R² avec R = alkyle ou aryle,
- A est un bras de liaison comportant au moins une double liaison covalente permettant la conjugaison de la fonction diazo avec le cycle aromatique et u est un nombre entier compris entre 0 et 2, préférentiellement de 0 ou 1,
- -Y-X- représente -CONH-, -NHCO-, -CH₂O-, -CH₂S-,
- -Z- représente -NH-, -NHCO-, -CONH- ou -O-,
- m est un nombre entier compris entre 1 et 10, préférentiellement entre 1 et 3, et
- p est un nombre entier compris entre 1 et 10, préférentiellement entre 1 et 3.

De plus pour que ce marquage soit encore plus efficace, il est également intéressant que les polynucléotides naturels ou de synthèse soient également fragmentés. La taille réduite de ces acides nucléiques les rend plus accessible pour le marquage. En ce qui concerne la fragmentation des acides nucléiques, de nombreuses méthodes sont décrites dans l'état de la technique. Premièrement, la fragmentation peut être enzymatique, c'est-à-dire que la fragmentation des acides nucléiques peut être réalisée par des nucléases (DNases ou RNases). On génère alors des fragments de petites tailles avec des extrémités 3'-OH, 5'-OH, 3'-phosphate, 5'-phosphate.

Deuxièmement, la fragmentation peut être chimique. Par exemple dans le cas des ADN, on peut effectuer la dépurination ou la dépyrimidination des ADN, qui sont alors fragmentés en présence d'une base par un mécanisme dit de "beta-élimination". La fragmentation des ADN peut être réalisée par des mécanismes d'oxydation, d'alkylation, d'addition de radicaux libres entre autres.

Pour fragmenter les ARN ou des ADN, comme cela est décrit respectivement dans nos brevet US-A-6,376,179 et demande de brevet WO-A-01/44507, on utilise des cations métalliques souvent associés à des molécules organiques utilisées comme catalyseurs chimiques, par exemple l'imidazole. Cette fragmentation est préférentiellement réalisée en milieu alcalin et génère des fragments avec des extrémités 3'-phosphate. Dans ce cas, la fixation d'un marqueur se fait au niveau du seul phosphate, d'un fragment d'acide nucléique, libéré lors de la coupure. Il n'y a aucune spécificité, la fragmentation pouvant être réalisée sur n'importe quel type d'acide nucléique et ce de manière aléatoire. En fait, notre procédé permet par exemple la préparation de sonde de détection. Enfin, le phosphate n'est qu'un bras de liaison entre l'acide nucléique et le marqueur.

Par "marqueur detectable", on entend au moins un marqueur capable de générer directement ou indirectement un signal détectable. Une liste non limitative de ces marqueurs suit :
- les enzymes qui produisent un signal détectable par exemple par colorimétrie, fluorescence, luminescence, comme la péroxydase de raifort, la phosphatase alcaline, la β-galactosidase, la glucose-6-phosphate déshydrogénase,
- les chromophores comme les composés fluorescents, luminescents, colorants,
- les groupements à densité électronique détectable par microscopie électronique ou par leur propriété électrique comme la conductivité, l'ampérométrie, la voltamétrie, l'impédance,
- les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel,
- les molécules radioactives comme le ³²P, le ³⁵S ou le ¹²⁵I.

De préférence, le marqueur n'est pas un marqueur radioactif pour éviter les problèmes de sécurité liés à ces marqueurs.

Par exemple le marqueur peut être un composé fluorescent de faible encombrement stérique comme la fluorescéine, le dansyl, les chromophores du type IR (Li-COR Inc, Lincoln NE, USA), des dérivés cyanines comme le Cy5 et le Cy3 (Randolph J.B. and al, Nucleic Acids Res., 25(14), p2923-2929, 1997) et en particulier les dérivés du Cy5 ou bien le traceur est un haptène de faible encombrement stérique comme la biotine ou un dérivé de l'abiétane (voir la demande WO-A-00/07982). Par faible encombrement stérique, on entend un poids moléculaire inférieur à 2000 g/mole (par exemple, la bis-bioPDAM étant d'un poids de 1064 g/mole). Dans le cas d'un fluorophore, il est préférable de travailler avec des fluorophores dont la longueur d'onde d'excitation est supérieure à 450 nm, de préférence supérieure à 600 nm.

Dans le cas où le traceur est un haptène qui ne produit pas de signal par lui-même comme par exemple la biotine, la détection est réalisé par la reconnaissance d'un anti-ligand marqué comme décrit plus haut. Dans le cas de la biotine, on utilise de préférence de la streptavidine ou un anticorps anti-biotine couplé à un composé fluorescent comme la fluorescéine, Cy5 ou la phycoérythrine. Dans le cas de l'abiétane, on utilise un anticorps monoclonal comme décrit dans la demande de brevet WO-A-00/07982.

C'est ce que l'on appelle des précurseurs de marquage. Par "précurseur de marquage", on entend un composé ayant au moins une fonction diazo réactive éventuellement protégée différente de la fonction diazométhyle et compatible avec ladite fonction qui permet la fixation d'un marqueur ultérieurement, c'est-à-dire après l'une quelconque des étapes du procédé et en particulier après l'étape d'oxydation par MnO₂. En particulier, la précurseur de marquage peut comprendre le bras de liaison L, décrit dans les formules chimiques ci-dessus. Un exemple de stratégie utilisant un précurseur de marqueur est souvent associé au cas de l'amplification de signal mais d'autres variantes sont possibles en utilisant les différents groupements protecteurs qui sont bien connus de l'homme du métier.

Des systèmes indirects peuvent aussi être utilisés, comme par exemple des ligands capables de réagir avec un anti-ligand.

Les couples ligand/anti-ligand sont bien connus de l'homme du métier, ce qui est le cas par exemple des couples suivants :
- biotine/streptavidine,
- haptène/anticorps,
- antigène/anticorps,
- peptide/anticorps,
- sucre/lectine,
- polynucléotide/complémentaire du polynucléotide.

Dans ce cas, c'est le ligand qui porte l'agent de liaison. L'anti-ligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal, par exemple par l'utilisation de structure multimérique. Cette technique d'amplification du signal est bien connue de l'homme du métier, et l'on pourra se reporter aux demandes de brevet antérieures FR98/10084 ou WO-A-95/08000 de la demanderesse ou à l'article J. Histochem. Cytochem. 45 : 481-491, 1997.

Par "structure multimérique", on entend un polymère formé d'unités répétées de synthons chimiques ou biologiques. De nombreuses variantes de telles structures utilisables dans cette publication sont connues, comme par exemple :
- les polymères linéaires (EP-A-0.561.722, EP-A-0.669.991),
- les polymères ramifiés (WO-A-01/92361),
- les particules (EP-A-0 827 552),
- les dendrimères (US-A-4,507,466 ; US-A-4,568,737 ; US-A-6,083,708),
- les polynucléotides, et
- les polypeptides.

Un autre exemple de systèmes indirects utilise une liaison covalente spécifique entre le ligand et l'anti-ligand par exemple méthylcétone et alcoxyamine. Des exemples de ce système sont décrits dans les demandes de brevet WO-A-00/40590 et WO-A-98/05766. Ces systèmes de détection indirects peuvent conduire, dans certaines conditions, à une amplification du signal et l'on pourra se reporter aux demandes de brevet antérieures WO-A-00/07982, WO-A-01/92361 et WO-A-95/08000 pour des exemples d'amplification chimique en utilisant des polymères ou à la demande WO-A-01/44506 pour les systèmes d'amplification chimique par empilement.

Dans un mode particulier de l'amplification de signal, au moins deux marqueurs sont présents sur le réactif de marquage.

De plus les réactifs de marquage de l'invention sont solubles dans des solvants polaires et miscibles à l'eau comme le DMF, le DMSO, CH₃CN, THF, DMA (diméthylacétamide), NMP (N-méthylpyrrolidone), DME (diméthoxyéthane).

De préférence, les réactifs de marquage sont solubles dans le DMSO ou l'eau.

Par solvant miscible à l'eau, on entend un solvant qui est miscible dans une proportion d'au moins 5% en volume avec de l'eau ou un tampon aqueux contenant des sels.

Par "molécule biologique", on entend un composé qui possède au moins un site de reconnaissance lui permettant de réagir avec une molécule cible d'intérêt biologique. A titre d'exemple on peut citer comme molécules biologiques les acides nucléiques, les antigènes, les anticorps, les polypeptides, les protéines, les haptènes. Pour l'invention, les molécules biologiques sont des acides nucléiques.

Le terme "acide nucléique" signifie un enchaînement d'au moins deux désoxyribonucléotides ou ribonucléotides comprenant éventuellement au moins un nucléotide modifié, par exemple au moins un nucléotide comportant une base modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Ce polynucléotide peut aussi être modifié au niveau de la liaison internucléotidique comme par exemple les phosphorothioates, les H-phosphonates, les alkyl-phosphonates, au niveau du squelette comme par exemple les alpha-oligonucléotides (FR-A-2 607 507) ou les PNA (M. Egholm et al., J. Am. Chem. Soc., 114, 1895-1897, 1992 ou les 2' O-alkyl ribonucléotides. L'acide nucléique peut être naturel ou synthétique, un oligonucléotide, un polynucléotide, un fragment d'acide nucléique, un ARN ribosomique, un ARN messager, un ARN de transfert, un acide nucléique obtenu par une technique d'amplification enzymatique telle que :
- PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
- LCR (Ligase Chain Reaction), exposée par exemple dans la demande de brevet EP-A-0.201.184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevet US-A-5,399,491.

On parle alors d'amplicons pour désigner les acides nucléiques générés par une technique d'amplification enzymatique.

Chacune de ces modifications peut être prise en combinaison pour peu qu'au moins un phosphate soit présent dans l'acide nucléique.

Le terme "haptène" désigne des composés non immunogènes, c'est-à-dire incapables par eux-mêmes de promouvoir une réaction immunitaire par production d'anticorps, mais capables d'être reconnues par des anticorps obtenus par immunisation d'animaux dans des conditions connues, en particulier par immunisation avec un conjugué haptène-protéine. Ces composés ont généralement une masse moléculaire inférieure à 3000 Da, et le plus souvent inférieure à 2000 Da et peuvent être par exemple des peptides glycosylés, des métabolites, des vitamines, des hormones, des prostaglandines, des toxines ou divers médicaments, les nucléosides et nucléotides.

Par "étape de purification", on entend notamment la séparation entre les acides nucléiques des micro-organismes et les constituants cellulaires relargués dans l'étape de lyse qui précéde la purification des acides nucléiques. Ces étapes de lyse sont bien connues à titre d'exemple indicatif, on peut utiliser les méthodes de lyse telles que décrite dans les demandes de brevet :
- WO-A-00/60049 sur la lyse par sonication,
- WO-A-00/05338 sur la lyse mixte magnétique et mécanique,
- WO-A-99/53304 sur la lyse électrique, et
- WO-A-99/15621 sur la lyse mécanique.

L'homme du métier pourra utiliser d'autres méthodes de lyse bien connues telles que les chocs thermiques ou osmotiques ou les traitements par des agents chaotropiques, tels que les sels de guanidium, notamment décrit dans le brevet US-A-5,234,809, appartenant à la Demanderesse.

Cette étape permet généralement de concentrer les acides nucléiques. A titre d'exemple, on peut utiliser des particules magnétiques (voir à ce sujet les brevets de la Demanderesse : US-A-4,672,040 et US-A-5,750,338), et ainsi purifier les acides nucléiques, qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO-A-97/45202 et WO-A-99/35500.

Le terme "support solide" tel qu'utilisé ici inclut tous les matériaux sur lesquels peuvent être fixées des molécules portant la fonction diazométhyl ou une fonction dérivée de celle-ci, telle que des produits de dégradation ou de réarrangement du diazo, azines par exemple, libérés pendant la réaction de marquage et qui ont besoin d'être éliminés. Des matériaux de synthèse ou des matériaux naturels, éventuellement modifiés chimiquement, peuvent être utilisés comme support solide :
- notamment les polysaccharides, tels que les matériaux à base de cellulose, par exemple du papier, des dérivés de cellulose tels que l'acétate de cellulose et la nitrocellulose, ou le dextran,
- notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels, etc. Le support solide peut être sous la forme d'une plaque de microtitration, d'une membrane, d'une particule ou d'une plaque sensiblement plane de verre ou silicium ou dérivés. Ces supports peuvent être également fonctionnalisés par des groupements d'intérêt, anioniques et/ou acides comme cela sera expliqué ultérieurement, pouvant réagir avec la fonction diazo ou des fonctions dérivées de celle-ci. Pour l'invention, des molecules de capture portant des fonctions anioniques et/ou acides sont immobilisé sur le support solide.

Comme évoqué précédemment, l'art antérieur décrit également sur le support l'utilisation de composés portant des fonctions diazo dans le but de marquer les acides nucléiques, par liaison sur leurs groupements phosphates, dans le but de les rendre détectables, notamment par lecture de fluorescence. Dans ce cas, le composé diazo porte un groupement fluorescent (Fluorescéine, Cy5) ou un groupement (type biotine, haptène ou fonction réactive) permettant la liaison covalente ou non-covalente avec une molécule fluorescente.

L'art antérieur décrit l'utilisation de nombreux moyens de purification liés aux processus de marquage. Parmi ces moyens, on peut citer l'extraction par une phase organique, la filtration et la gel filtration. Cependant la technique de référence est l'utilisation de la silice, sous forme de poudre, de gel ou de particules magnétiques, pour purifier les acides nucléiques avant ou après marquage, dans un processus aboutissant à une détection par hybridation spécifique (concernant les puces à ADN, mais aussi les plaques ELOSA ou des formes de tests rapides). La purification avant marquage permet d'améliorer considérablement le rendement de marquage, la purification post-marquage permet d'améliorer de façon déterminante le rendement d'hybridation et donc le ratio signal/bruit, nécessaire à une bonne sensibilité du test. En effet, l'excès du marqueur utilisé pendant le marquage et n'ayant pas réagi peut affecter considérablement l'hybridation.

La purification sur silice impose une étape de lavage et d'élution de la phase solide. La présence de trois étapes (fixation/lavage/élution) impose donc des transferts de liquides, potentiellement facteurs de contamination, de perte de matériel, et d'une façon générale, modérément automatisable.

De plus, ce processus impose l'intervention d'un opérateur durant toute la durée de l'opération, et l'utilisation de sels chaotropiques (irritants).

Pour résumer, le problème essentiel avec l'ensemble de ces procédés de marquage avec étape de purification réside dans le fait que, pour que le marquage soit réellement efficace, il est nécessaire d'utiliser un excès de marqueurs afin que tous les acides nucléiques aient la possibilité d'être marqués. La présence dans la solution liquide traitée d'acides nucléiques marqués mais également de marqueurs n'ayant pas réagi implique que l'échantillon liquide ne peut ensuite être utilisé dans une étape de détection spécifique. Préalablement il y a lieu d'éliminer les marqueurs présents en excès. Pour ce faire, il est possible de fixer les acides nucléiques sur des particules de silice, contenant éventuellement un matériau magnétique afin d'immobiliser les acides nucléiques, marqués ou non, et de procéder à leur lavage par élimination des constituants indésirables. Le brevet US-A-5,234,809 de la Demanderesse, déjà mentionné plus haut, permet ce genre de procédé. Toutefois ceci engendre des étapes supplémentaires qui sont onéreuses en temps de travail des praticiens et en materiel.

La présente invention consiste donc à utiliser les caractéristiques chimiques d'un marqueur ou d'un précurseur de marquage afin de permettre sa fixation préférentiellement sur un acide nucléique d'intérêt et éventuellement, si une telle fixation n'est pas intervenue de permettre la capture de celui-ci sur une phase solide n'entraînant aucune phase de lavage. De plus les agents chaotropiques, irritants, ne sont pas nécessaires au processus de purification. De même, le procédé selon l'invention ne nécessite aucune système supplémentaire de type pompe ou centrifugeuse, destiné à faire passer le liquide à travers la phase solide. Le processus peut nécessiter un système d'aimantation ou de filtration, si la phase de purification est composée de particules (magnétiques ou non), cependant ce dispositif n'est pas obligatoire et un processus totalement passif (passage du milieu de marquage sur la phase solide) peut être utilisé. Enfin le procédé est facilement automatisable, en raison de l'utilisation de phases solides, et de sa simplicité (mise en contact, incubation, passage en hybridation). Le procédé peut être utilisé dans un système utilisant un flux continu, permettant de simplifier l'intégration dans un dispositif autonome, type "Lab on Card", ou complètement intégré dans un seul tube, de la purification de l'acide nucléique à l'hybridation sur puce. Cela implique la réduction des risques de contamination, et la diminution du nombre de consommables employés.

A cet effet, la présente invention concerne, selon un premier mode de réalisation, un procédé de marquage d'acides nucléiques d'intérêt contenues dans un échantillon biologique, consistant à
a) disposer d'un récipient réactionnel,
b) immobiliser sur tout ou partie de la surface intérieure du récipient ou d'un support solide introduit dans ce récipient, des molécules de capture portant des fonctions anioniques et/ou acides, pouvant fixer un marqueur ou précurseur de marquage des acides nucléiques d'intérêt,
c) introduire dans ledit récipient réactionnel l'échantillon biologique mais également
   1) au moins un marqueur ou précurseur de marquage des acides nucléiques d'intérêt comprenant une fonction
      diazo, et
   2) éventuellement tout ingrédient nécessaire au marquage ou pré-marquage des acides nucléiques
      d'intérêt,
d) incuber le contenu du récipient réactionnel,
e) immobiliser le marqueur ou précurseur de marquage n'ayant pas réagi avec les acides nucléiques d'intérêt par réaction de la fonction diazo avec les fonctions anioniques et/ou acides des molécules de capture pour former une liaison covalente, et
f) utiliser les acides nucléiques d'intérêt marquées, c'est-à-dire celles ayant réagi avec ledit marqueur ou précurseur de marquage, pour des étapes ultérieures.

Selon un second mode de réalisation, l'invention concerne également un procédé de traitement d'un échantillon biologique contenant un mélange de acides nucléiques d'intérêt et d'au moins un marqueur ou précurseur de marquage des acides nucléiques d'intérêt comprenant une fonction diazo, éventuellement associé à tout ingrédient nécessaire au marquage des acides nucléiques d'intérêt, consistant à
a) disposer d'un récipient réactionnel,
b) immobiliser sur tout ou partie de la surface intérieure du récipient ou d'un support solide introduit dans ce récipient, des molécules de capture portant des fonctions anioniques et/ou acides pouvant fixer le marqueur ou précurseur de marquage des acides nucléiques d'intérêt,
c) introduire dans ledit récipient réactionnel l'échantillon biologique,
d) incuber le contenu du récipient réactionnel,
e) immobiliser le marqueur ou précurseur de marquage n'ayant pas réagi avec les acides nucléiques d'intérêt par fixation sur les molécules de capture, la fixation du marqueur ou précurseur de marquage n'ayant pas réagi avec les acides nucléiques d'intérêt, sur les molécules de capture s'effectuant par une liaison covalente, et
f) utiliser les acides nucléiques d'intérêt marquées, c'est-à-dire celles ayant réagi avec ledit marqueur ou précurseur de marquage, pour des étapes ultérieures.

Dans les deux cas de figure ci-dessus décrits la fixation du marqueur ou précurseur de marquage, n'ayant pas réagi avec les molécules biologiques d'intérêt, sur les molécules de capture s'effectue par une liaison covalente.

Dans un mode particulier de réalisation, et préalablement à l'étape a) ci-dessus décrite, l'échantillon biologique est traité selon au moins l'une des étapes suivantes :
- le transfert depuis un autre récipient réactionnel amont,
- la lyse d'un matériel biologique complexe pour rendre les molécules biologiques d'intérêt accessibles et/ou détectables,
- la capture ou isolement des molécules biologiques d'intérêt, et/ou
- le traitement des molécules biologique d'intérêt afin de rendre leur détection possible ou d'améliorer leur détection ; ce traitement peut consister, par exemple, en un traitement thermique.

Dans un autre mode particulier de réalisation, l'étape f) précédemment décrite est suivie d'au moins une étape ultérieure suivante :
- le transfert vers un autre récipient réactionnel aval,
- le marquage des molécules biologiques d'intérêt pré-marquées,
- la purification des molécules biologiques d'intérêt marquées ou pré-marquées, et/ou
- la détection des molécules biologiques d'intérêt marquées et hybridées sur des sondes de capture.

La détection des molécules biologique d'intérêt peut par exemple être réalisée de manière intéressante en phase homogène, avec ou sans hybridation avec une molécule de détection (par exemple une sonde nucléique).

Dans tous les cas de figure précédent, la surface intérieure du récipient ou le support solide introduit dans ce récipient portent des fonctions anioniques et/ou acides, et le marqueur ou précurseur de marquage comprend une fonction diazo (-N=N).

Dans ce dernier mode de réalisation, les fonctions portées par la surface intérieure du récipient ou le support solide introduit dans ce récipient sont constituées par des fonctions carboxyliques et/ou sulfoniques.

Dans tous les cas de figure, les molécules biologiques d'intérêt sont constituées par des acides nucléiques et/ou des fragments d'acides nucléiques.

Dans ce dernier mode de réalisation, les acides nucléiques et/ou les fragments d'acides nucléiques sont constitués par de l'ADN, de l'ARN, des polymères chimériques d'ADN-ARN, pouvant optionnellement contenir au moins un nucléotide thiophosphate, un LNA, un 2'-O-Me et/ou un dérivé méthylphosphonates. Dans l'invention où les molécules biologiques d'intérêt sont des acides nucléiques ou des fragments d'acide nucléique, préalablement à l'étape a), définie ci-dessus, l'échantillon biologique est traité selon au moins l'une des étapes suivantes :
- le transfert depuis un autre récipient réactionnel amont,
- la lyse du matériel biologique complexe, contenu par l'échantillon biologique, pour rendre les acides nucléiques accessibles,
- l'extraction des acides nucléiques à partir du matériel biologique complexe,
- l'amplification spécifique des acides nucléiques d'intérêt,
- la fragmentation desdits acides nucléiques d'intérêt ou amplicons, et/ou
- la transcription, ou la retrotranscription d'un acide nucléique d'intérêt, sans phénomène d'amplification notable.

Toujours dans le même cas de figure, l'étape f), définie préalablement, est suivie d'au moins une étape ultérieure suivante :
- le transfert vers un autre récipient réactionnel aval,
- le marquage des acides nucléiques pré-marqués,
- la purification des acides nucléiques marqués ou pré-marqués,
- la détection des acides nucléiques marqués ou pré-marqués, hybridés sur des sondes de capture,
- la transcription, ou la retrotranscription d'un acide nucléique d'intérêt, sans phénomène d'amplification notable, et/ou
- la détection en phase homogène des acides nucléiques marqués ou pré-marqués, avec ou sans utilisation de sondes de détection.

Selon un autre mode de réalisation de l'invention, les molécules de capture sont présentes en excès par rapport aux marqueurs, et que les marqueurs sont présents en excès par rapport aux acides nucléiques d'intérêt qui vont être marquées.

Plus particulièrement, les molécules de capture sont présentes en excès par rapport aux marqueurs libres ne réagissant pas avec les acides nucléiques, et que les marqueurs sont présents en excès par rapport aux acides nucléiques qui vont être marqués.

Pour permettre la détection et/ou la quantification et/ou la purification de l'acide nucléique d'intérêt, l'acide nucléique marqué est capable de former un complexe suffisamment complémentaire de la cible pour s'hybrider spécifiquement en fonction des conditions de réaction, et notamment de la température ou de la salinité du milieu réactionnel.

De son côté, sans toutefois que cette réaction soit spécifique, les marqueurs, n'ayant pas réagi ou marqueurs libres, conservent leur fonction réactive intact et sont donc aptes à réagir ultérieurement avec les molécules de capture qui elles-mêmes portent une fonction réactive complémentaire. La fonction réactive et la fonction réactive complémentaire forment donc une liaison covalente, ce qui permet l'immobilisation des marqueurs libres, l'échantillon biologique ne contenant alors plus que des marqueurs associés à des acides nucléiques.

Les figures ci-jointes montrent les différentes étapes du procédé de purification selon l'invention. Ils représentent un mode particulier de réalisation et ne peuvent pas être considérés comme limitant la portée de la présente invention.

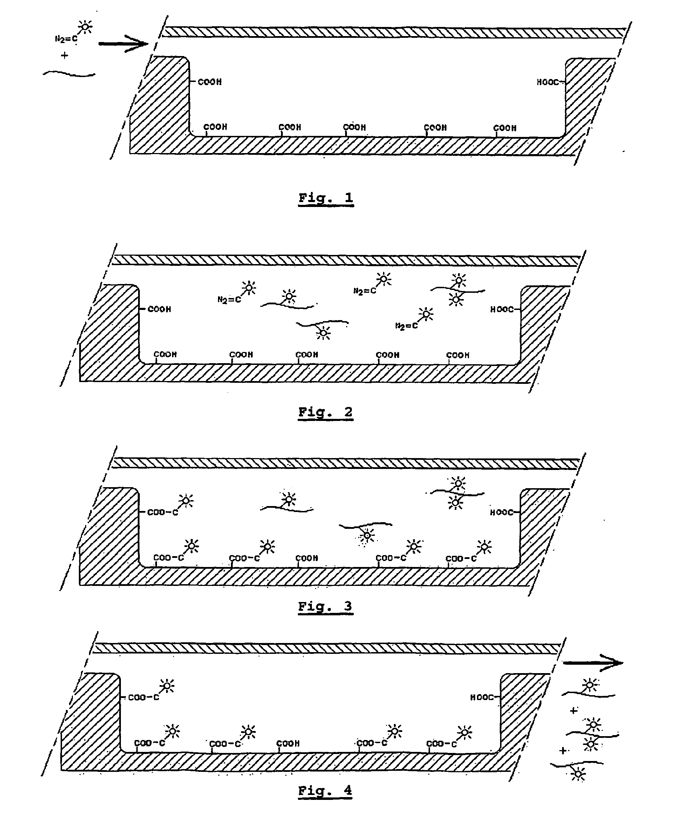

La figure 1 représente une vue en coupe transversale d'un récipient réactionnel dans lequel le procédé de purification est réalisé. Ce récipient comporte un couvercle supérieur et un corps inférieur évidé pour créer une espace où le procédé, selon l'invention, peut être réalisé. Cet espace comporte un canal d'amenée sur la gauche permettant l'entrée de l'échantillon biologique à traiter et un canal de décharge sur la droite permettant la sortie dudit échantillon biologique traité. L'espace intérieur du récipient est traité ou est revêtu pour exhiber des molécules de capture portant des fonctions réactives carboxyliques. L'espace intérieur du couvercle pourrait également subir le traitement. Comme évoqué précédemment, le canal d'amenée permet l'introduction selon la flèche d'un échantillon biologique liquide, contenant, entre autres, des acides nucléiques et des marqueurs, portant une fonction diazo. Dans le cas représenté sur cette figure, les acides nucléiques ne sont pas encore marqués, mais il est également possible qu'ils puissent être mis préalablement en contact avec les marqueurs.
La figure 2 représente une vue identique à la figure 1, dans laquelle l'échantillon biologique présent dans l'espace intérieur du récipient réactionnel contient des acides nucléiques marqués et des marqueurs libres, c'est-à-dire n'ayant pas réagi avec les acides nucléiques. Du fait que les marqueurs sont présents en excès par rapport aux acides nucléiques, tous ces acides nucléiques seront marqués, la contrepartie étant que les marqueurs libres sont nombreux.
La figure 3 est toujours identique aux figures 1 et 2, mais on remarque qu'après un certain laps de temps, soit en général dans le cas du bis-BioPDAM, d'au moins une dizaine de minutes mais inférieur à une heure, les marqueurs libres sont majoritairement liés aux fonctions réactives carboxyliques présentes dans l'espace intérieur du récipient. Par majoritairement on entend que les marqueurs libres sont capturés pour descendre en dessous de la concentration inhibitrice (inférieure à 2 mM). Il est bien évident que ces fonctions réactives sont présentes en excès par rapport aux marqueurs libres n'ayant pas réagi avec les acides nucléiques.
La figure 4 est toujours identique aux figures 1 à 3. Comme évoqué précédemment, le canal de sortie permet la sortie selon la flèche à droite de la figure de l'échantillon biologique liquide traité, contenant, entre autres, des acides nucléiques marqués mais pas de marqueurs libres.

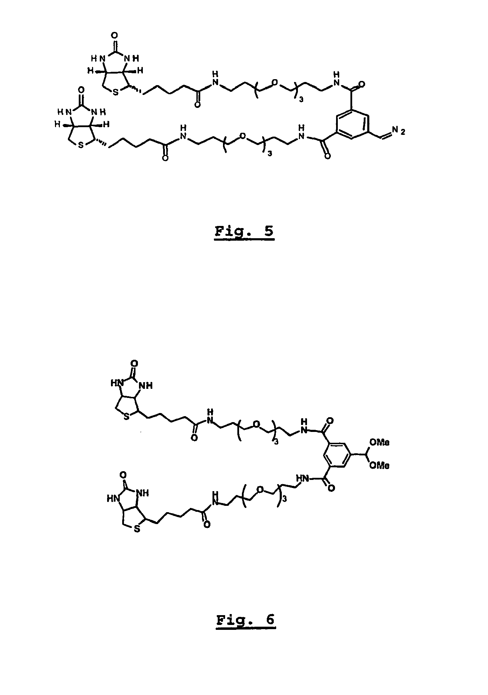

La figure 5 représente la formule développée de la molécule N,N'-Bis(13-biotinoylamino-4,7,10-trioxatridécyl)-5-(diazométhyl)isophtalamide, également appelée par la suite "bis-BioPDAM" pour faciliter la compréhgension du texte.

Enfin, la figure 6 représente la formule développée de la molécule N,N'-Bis(13-biotinoylamino-4,7,10-trioxatridécyl)-5-(diméthoxyméthyl)isophtalamide, également appelée par la suite "acétal" pour faciliter la compréhension du texte.

### Exemple 1 - Marquage et purification sur particules carboxyliques du produit d'amplification RT-PCR suivie d'une analyse sur puce à ADN (Affymetrix, Santa Clara, CA) :

Dans le présent exemple, la fixation des marqueurs libres ne s'effectue pas directement sur un support solide, mais il permet de contrôler la validité du concept de l'invention en testant la réactivité des marqueurs avec des particules magnétiques)

### A - PCR Influenza B :

Cette expérience est réalisée sur un modèle dit *"Influenza* B". Ce nom désigne les amplicons générés par RT-PCR à partir d'une séquence de 190 bases d'un fragment du gène de l'ARN du virus *Influenza B*.

Les conditions de la préparation des prélèvements, l'extraction des ARN viraux, leur amplification et la séquence des amorces est décrite dans l'article "Effectiveness of Reverse Transcription-PCR, Virus Isolation, ans Enzyme-linked Immunosorbent Assay for diagnosis of Influenza A Virus Infection in different Age Groups", Steininger C. et al., J. Clin. Microbiol., (2002); 40(6), 2051-2056.

La RT-PCR est effectuée en utilisant comme matrice de départ des préparations d'ARN viral (10³ copies par amplification) avec le kit Titan One Tube RT-PCR System (Roche Diagnostic Corporation, Basel, Suisse, Référence : 11 855 476 001) avec 0,2 mM de chaque désoxyribonucléotide, 0,3 µM d'amorces et 0,4 µL d'enzyme.

Les paramètres du cycle de la RT-PCR sont les suivants : 60°C pendant 30 minutes pour réaliser la réaction de Reverse Transcription, puis 40 cycles selon le protocole suivant : 94°C pendant 20 secondes, puis 50°C pendant 30 secondes et enfin 72°C pendant 30 secondes, puis 4°C jusqu'à l'arrêt du thermocycler.

Les amplicons issus de la RT-PCR décrite ci-dessus sont désignés par la suite par les termes "PCR *Influenza* B".

### B - Marquage en phase homogène (protocole de référence) :

Le marquage en phase homogène est utilisé comme technique de référence ou de "contrôle" par rapport à l'invention.

Le protocole a été mis au point en utilisant une concentration finale de marqueur suffisante (2 mM) pour permettre un marquage suffisant de l'ADN, mais inférieur au seuil à partir duquel le marqueur libre affecte l'hybridation.

Un volume de 5 µL de PCR *Influenza* B, est mélangé à 5 µL de N,N'-Bis(13-biotinoylamino-4,7,10-trioxatridécyl)-5-(diazométhyl)isophtalamide, ci-après référencé "bis-BioPDAM", voir figure 5, dilué à 6 mM dans du DiMéthyle SulfOxyde, ci-après désigné par "DMSO", et 5 µL d'H₂O, puis incubé 10 minutes à 80°C.

Le milieu réactionnel est ensuite mis en contact avec la puce à ADN (Affymetrix, Santa Clara, CA) pour l'étape d'hybridation en utilisant le protocole du fournisseur. La puce à ADN utilisée est conçue pour l'analyse de la région amplifiée lors de la RT-PCR. Une description du protocole d'hybridation ainsi qu'une description des technologies utilisées pour l'analyse des résultats est décrite par A. Troesch et al. : "Mycobacterium species identification and rifampin resistance testing with high-density DNA probe arrays" J. Clin. Microbiol. (1999), 37, 49-55.

La lecture de la fluorescence émise à la surface de la puce à ADN après marquage et hybridation, ainsi que la génération des données en termes d'intensité du signal et du pourcentage d'homologie, sont réalisées par les systèmes de lecture et le logiciel fournit par les Genechip^{®} Instrument system et Genechip Information System^{®} (Affymetrix, Santa Clara, CA).

Le système de lecture fournit des intensités en signal et bruit de fond exprimés en RFU ("Relative Fluorecent Units"). Le pourcentage d'homologie est donné par rapport à une séquence de référence (correspondant à la séquence de la cible amplifiée, obtenue par séquençage).

Les résultats en terme d'intensité médianne du signal (I), du bruit de fond (B) et du pourcentage d'homologie (% Homologie) sont rassemblés dans un tableau de résultat.

De manière générale, on considère qu'un pourcentage d'homologie supérieur à 90% est un résultat satisfaisant, bien que l'on cherche en général un résultat supérieur à 95%. Une intensité élevée avec un bruit de fond faible (Rapport I/B élevé) est le deuxième résultat recherché dans les mises au point.

### C - Marquage en phase homogène à concentration de marqueur inhibitrice (contrôle) :

Dans cette expérience, une concentration finale du marqueur bis-BioPDAM plus importante est utilisée (5 mM). Cette concentration affecte l'hybridation en l'absence de purification, mais permet d'obtenir un signal supérieur (en raison d'un rendement de marquage plus important) lorsque ce même milieu réactionnel est purifié efficacement.

Un volume de 5 µL de PCR Influenza B est mélangé à 5 µL de bis-BioPDAM, dilué à 15 mM dans du DMSO, et 5 µL d'H₂O, puis incubés 10 minutes à 80°C.

Le milieu réactionnel est ensuite mis en contact avec la puce pour l'hybridation et la détection selon le protocole décrit au paragraphe B ci-dessus.

### D - Marquage en phase homogène avec purification sur membrane de silice (contrôle) :

Dans cette expérience, nous avons utilisé des colonnes de purification d'acides nucléiques contenant une membrane de silice commercialisées par QIAgen Gmbh (Hilden, Allemagne). Cette technique constitue la méthode de référence. Dans ce cas, le protocole de marquage a été réalisé en utilisant une concentration finale de bis-BioPDAM plus importante (5 mM), concentration de marqueur qui affecte l'hybridation sur puce en l'absence de purification efficace.

Un volume de 5 µL de PCR *Influenza* B est mélangé à 5 µL de bis-BioPDAM, dilué à 15 mM dans du DMSO, et 5 µL d'H₂O, puis incubés 10 minutes à 80°C.

Le milieu réactionnel est ensuite purifié par le kit QIAQuick (QIAgen GmbH, Hilden, Allemagne. Référence : 28 306) en utilisant le protocole préconisé par le fournisseur, puis il est mis en contact avec la puce à ADN pour l'hybridation selon le protocole décrit au paragraphe B déjà décrit.

### E - Marquage en phase homogène avec purification sur particules carboxyliques (invention) :

Dans cette expérience, un produit de marquage, obtenu en utilisant une concentration finale de bis-BioPDAM plus importante (5 mM) est purifié avec une phase solide carboxylique (invention). La concentration de marqueur de 5 mM affecte l'hybridation en l'absence d'une étape de purification efficace. C'est donc la concentration retenue pour étudier l'efficacité de la purification.

Un volume de 5 µL de PCR *Influenza* B est mélangé à 5 µL de bis-BioPDAM, dilué à 15 mM dans du DMSO, et 5 µL d'H₂O, puis incubé 10 minutes à 80°C.

Le milieu réactionnel est ensuite incubé 10 minutes à température ambiante en utilisant 2,5 mg de Standard Carboxyl-Adembeads (Référence: 0213, Ademtech, Pessac, France, ci-après référencé "particules carboxyliques"), les particules sont ensuite séparées par aimantation du milieu réactionnel, et celui-ci est mis en contact avec la puce à ADN pour l'étape d'hybridation selon le protocole décrit plus haut au paragraphe B.

### F - Résultats et conclusion :

Les résultats en terme de pourcentage d'homologie, d'intensité du signal (I) et de bruit de fond (B) sont regroupés dans le tableau 1 suivant :

**Tableau 1 : Etude comparative du procédé utilisant l'invention par rapport aux contrôles sans purification ou avec purification sur membrane de silice.**

| **Procédé de marquage** | % Homologie | I | B | I/B |
|---|---|---|---|---|
| **B - Marquage en phase homogène (référence)** | 93,1 | 1699 | 308 | 5,5 |
| **C - Marquage en phase homogène à concentration de marqueur (5mM) affectant l'hybridation (contrôle):** | 47,5 | 135 | 339 | 0,3 |
| **D - Marquage en phase homogène avec purification sur mlembrane de silice (contrôle):** | 97,9 | 5905 | 297 | 19,9 |
| **E - Marquage en phase homogène avec purification sur particules carboxyliques (invention)** | 97,9 | 8167 | 434 | 18,7 |

Les résultats sont exprimés en pourcentage d'homologie, en intensité du signal (I) et en bruit de fond (B)

En conclusion, les résultats obtenus avec le procédé de référence sont sensiblement identiques à ceux obtenus avec la purification sur membrane de silice. D'autre part, on observe que les résultats obtenus en utilisant la "purification carboxylique" sont supérieurs en terme d'intensité (I) à ceux obtenus sans purification, ou en utilisant une purification sur membrane de silice Une augmentation du signal sur puce permet d'avoir un test plus robuste, moins dépendant de facteurs impactant localement le bruit de fond (poussières, précipités apparaissant sur puce, par exemple). D'autre part, la facilité opératoire du protocole de purification allège considérablement le process manipulatoire.

### Exemple 2 : Marquage et purification sur particules carboxyliques du produit d'amplification RT-PCR marqué et contaminé par un équivalent non réactionnel du marqueur :

Cet exemple sert de démonstration que la liaison qui se crée entre le support solide et la molécule de marquage est bien le fait d'une capture covalente du marqueur et non pas de son adsorption sur ledit support.

### A - But :

Dans cette expérience, un produit de marquage purifié est contaminé avec un intermédiaire de synthèse du bis-BioPDAM, le N,N'-Bis(13-biotinoylamino-4,7,10-trioxatridécyl)-5-(diméthoxyméthyl)isophtalamide, ci-après référencé "acétal", figure 6. Ce réactif ne porte pas la fonction DiAzoMéthyl, et ne peut donc réagir avec les fonctions carboxyliques.

Il présente par contre l'intégralité de la structure de la bis-BioPDAM, et peut donc s'adsorber aux surfaces de façon similaire à la bis-BioPDAM, dans le cas où les phénomènes de purification observés sont des phénomènes d'adsorption non spécifique, au lieu de phénomènes mettant en jeu des liaisons spécifiques.

L'acétal affecte l'hybridation des acides nucléiques de la même façon que le bis-bioPDAM, ce qui permet de juger de la qualité de son élimination.

### B - Expérience :

Un volume de 5 µL de PCR *Influenza* B est mélangé à 5 µL de bis-BioPDAM, dilué à 15 mM dans du DMSO, et 5 µL d'H₂O, puis incubé 10 minutes à 80°C. Le milieu réactionnel est ensuite purifié à l'aide du kit QIAQuick (QIAgen GmbH, Hilden, Allemagne. Référence : 28 306) en utilisant le protocole du fournisseur.

Cette préparation est réalisée en parallèle sur plusieurs aliquots d'un échantillon ayant subi une amplification par RT-PCR, afin d'avoir un volume de produit purifié suffisant pour réaliser plusieurs contrôles. Les produits de purification sont mélangés, afin de supprimer les variations induites par la préparation dudit échantillon

Des fractions de 15 µl, correspondant au volume d'éluât obtenu après purification par la purification sur membrane de silice du mélange, sont ensuite traitées de la manière suivante :
a) Hybridation directe sur puce Affymetrix comme décrit dans l'exemple 1/B (Référence).
b) Ajout de 5 mM d'acétal puis hybridation sur puce Affymetrix comme décrit dans l'exemple 1/B (Contrôle de l'inhibition par l'acétal).
c) Purification 10 minutes à température ambiante sur particules carboxyliques, telle quedécrite dans l'exemple 1/E puis hybridation sur puce Affymetrix comme décrit dans l'exemple 1/B (Contrôle de l'action des particules sur le produit de marquage).
d) Ajout de 5 mM d'acétal puis purification 10 minutes à température ambiante sur particules carboxyliques telle que décrite dans l'exemple 1/E et hybridation sur puce Affymetrix comme décrit dans l'exemple 1/B (contrôle de l'action des particules sur l'acétal).

### C - Résultats et conclusion :

Les résultats en terme de pourcentage d'homologie, d'intensité du signal (I) et de bruit de fond (B) sont regroupés dans le tableau 2 suivant :

**Tableau 2 : Etude comparative du procédé utilisant l'invention (C et D) par rapport à deux contrôles (A et B) ne l'utilisant pas, en présence d'un composé inhibiteur, ne pouvant réagir sur la fonction carboxylique (B et D)**

| **Procédé de marquage** | % Homologie | I | B | I/B |
|---|---|---|---|---|
| **A - Référence** | 93,1 | 1699 | 308 | 5,5 |
| **B - Contrôle de l'inhibition par l'acétal** | 39,5 | 127 | 260 | 0,5 |
| **C - Contrôle action particules carboxyliques sur marqueur** | 97,4 | 1911 | 302 | 6,3 |
| **D - Contrôle action particules carboxyliques sur acétal** | 50,0 | 525 | 369 | 1,4 |

Les résultats sont également exprimés en pourcentage d'homologie, en intensité du signal (I) et en bruit de fond (B).

En conclusion, le dérivé acétal affecte l'hybridation des acides nucléiques (B et D), qu'il y ait eu ou non de prétraitement avec des particules carboxyliques (D). Ce résultat indique qu'en l'absence de réaction avec la fonction carboxylique, l'acétal ne peut réagir. Il n'y a donc pas de suppression notable du composé acétal par adsorption. On peut alors conclure que la réaction DiaZoMéthyl-Acide carboxylique aboutissant à une liaison covalente est le principal mécanisme intervenant dans la purification du milieu réactionnel.

### Exemple 3 : Marquage et purification sur membrane carboxylique du produit d'amplification RT-PCR marqué :

### A - But :

Dans cette expérience, un produit de marquage, obtenu en utilisant une concentration finale de bis-BioPDAM de 5 mM, est purifié en utilisant une membrane portant des fonctions carboxyliques (Biodyne C, Référence S60314 Pall Gelman Sciences, New York, USA, ci-après référencé Biodyne C). La concentration de marqueur de 5 mM affecte l'hybridation en l'absence d'une étape de purification efficace. C'est donc la concentration retenue pour étudier l'efficacité de la purification.

### B - Expérience:

Un volume de 5 µL de PCR Influenza B est mélangé à 5 µL de bis-BioPDAM, dilué à 15 mM dans du DMSO, et 5 µL d'H₂O, puis incubé 10 minutes à 80°C. Cette expérience est réalisée en double, puis les produits de marquage sont mélangés et divisés en deux volumes égaux afin de limiter les effets néfastes potentiellement dus à une variabilité dans le protocole opératoire. Le milieu réactionnel est ensuite incubé 10 minutes à température ambiante en utilisant 6 mm² de Biodyne C (b), ou laissé à température ambiante (référence a), puis ce milieu ainsi traité est mis en contact avec la puce à ADN pour l'étape d'hybridation selon le protocole décrit dans l'exemple 1/B.

### C - Résultats et conclusion :

Les résultats en terme de pourcentage d'homologie, d'intensité du signal (I) et de bruit de fond (B) sont regroupés dans le tableau 3 suivant :

**Tableau 3 : Etude comparative d'un procédé utilisant l'invention (b)par rapport à une référence ne l'utilisant pas (a).**

| **Procédé de marquage** | % Homologie | I | B | I/B |
|---|---|---|---|---|
| **a Référence** | 94,7 | 4252 | 564 | 7,5 |
| **b - Purification par membrane carboxylique** | 94,7 | 23811 | 2174 | 11,0 |

Les résultats sont encore une fois exprimés en pourcentage d'homologie, en intensité du signal (I) et en bruit de fond (B).

En conclusion, la membrane Biodyne C, portant des fonctions carboxyliques, permet d'avoir une purification de l'échantillon de même nature qu'avec des particules magnétiques.

### Exemple 4 : Marquage et purification avec un polymère carboxylique du produit d'amplification RT-PCR marqué :

### A - But :

Dans cette expérience, un produit de marquage obtenu en utilisant une concentration finale de bis-BioPDAM de 5 mM est purifié en utilisant un polymère portant des fonctions carboxyliques (Poly-Acrylic Acid sodium salt) standard 28'000, Référence 81124, Fluka, Buchs, Suisse ; ci-après référencé APA). Comme décrit précédemment, la concentration de marqueur de 5 mM affecte l'hybridation en l'absence d'une étape de purification efficace.

### B- PCR Myco 16 S :

Cette expérience est réalisée sur un modèle dit "Myco 16S", désignant les amplicons générés par PCR à partir d'une séquence de 180 bases d'un fragment du gène codant pour l'ARN ribosomal 16S de *Mycobacterium tuberculosis.*

Les conditions pour la culture, l'extraction des mycobactéries ainsi que les amorces d'amplification sont données par A. Troesch et al. : "Mycobacterium species identification and rifampin resistance testing with high-density DNA probe arrays" J. Clin. Microbiol. (1999), 37, 49 - 55.

La PCR est effectuée en utilisant comme matrice de départ des préparations d'ADN génomique (10³ copies par PCR) avec le kit FastStart High Fidelity PCR System (Roche Diagnostic Corporation, Basel, Suisse, Référence : 03 553 426 001) avec 0,2 mM de chaque désoxyribonucléotide, 0,3 µM d'amorces et 0,4 µL d'enzyme.

Les paramètres du cycle de la PCR sont les suivants : 95°C pendant 4 minutes puis 35 cycles d'amplification selon le protocole suivant : 95°C pendant 30 secondes, puis 55°C pendant 30 secondes et enfin 72°C pendant 30 secondes. Enfin on maintient à 4°C jusqu'à l'arrêt du thermocycler, pour éviter une éventuelle dégradation des amplicons à température ambiante.

La solution contenant les amplicons issus de la PCR, décrite ci-dessus, est désignée par la suite par les termes "PCR 16S".

### C - Expérience:

Un volume de 5 µL de PCR 16S, est mélangé à 5 µL de bis-BioPDAM, dilué à 2,5 mM dans du DMSO, et 15 µL d'H₂O, puis incubé 10 minutes à 80°C. Cette expérience est réalisée en double, puis les produits de marquage sont mélangés et divisés en deux volumes égaux afin de limiter les effets néfastes potentiellement dû à une variabilité dans le protocole opératoire.

Le milieu réactionnel est ensuite incubé 10 minutes à température ambiante (a), ou avec un culot de 10 µl de solution à 20 % d'APA séchée sous vide d'air (b), puis il est mis en contact avec la puce à ADN pour l'étape d'hybridation selon le protocole décrit dans l'exemple 1/B.

### D - Résultats et conclusion :

Les résultats en terme de pourcentage d'homologie, d'intensité du signal (I) et de bruit de fond (B) sont regroupés dans le tableau 4 suivant :

**Tableau 4 : Etude comparative d'un procédé utilisant l'invention (b)par rapport à une référence ne l'utilisant pas (a).**

| **Procédé de marquage** | % Homologie | I | B | I/B |
|---|---|---|---|---|
| **a - Référence** | 97,9 | 2313 | 525 | 4,4 |
| **b - Purification par APA** | 94,4 | 8091 | 928 | 8,7 |

En conclusion, le polymère portant des fonctions carboxyliques permet d'avoir une purification de l'échantillon comparable à celle réalisée en utilisant des particules magnétiques.

### Exemple 5 : Marquage et purification avec un polymère sulfonique, du produit d'amplification RT-PCR marqué et contaminé par un équivalent non réactionel du marqueur :

### A - But:

Dans cette expérience, un produit de marquage, obtenu en utilisant une concentration finale de bis-BioPDAM de 5 mM, est purifié en utilisant un polymère portant des fonctions sulfoniques (Référence 29 256-7, Sigma-Aldricht, Saint-Louis ; MI, USA, ci-après référencé Nafion).

### B - Expérience:

Un volume de 5 µL de PCR 16S, est mélangé à 5 µL de bis-BioPDAM, dilué à 2,5 mM dans du DMSO, et 15 µL d'H₂O, puis incubé 10 minutes à 80°C. Cette expérience est réalisée en double, puis les produits de marquage sont mélangés et divisés en deux volumes égaux afin de limiter les effets néfastes potentiellement dus à une variabilité dans le protocole opératoire.

Le milieu réactionnel est ensuite incubé :
- 10 minutes à température ambiante (a) ou
- avec un culot de 10 µl de Nafion à 5% dans le méthanol, séché sous vide d'air (b),
puis il est mis en contact avec la puce à ADN pour l'étape d'hybridation selon le protocole décrit dans l'exemple 1/B.

### C - Résultats et conclusion :

Les résultats en terme de pourcentage d'homologie, d'intensité du signal (I) et de bruit de fond (B) sont regroupés dans le tableau 5 suivant :

**Tableau 5 : Etude comparative d'un procédé utilisant l'invention (b)par rapport à une référence ne l'utilisant pas (a).**

| **Procédé de marquage** | % Homologie | I | B | I/B |
|---|---|---|---|---|
| **a - Référence** | 97,9 | 2313 | 525 | 4,4 |
| **b - Purification par Nafion** | 97,2 | 8887 | 1002 | 8,9 |

Les résultats sont encore une fois exprimés en pourcentage d'homologie, en intensité du signal (I) et en bruit de fond (B).

En conclusion, le polymère portant des fonctions sulfoniques permet d'avoir une purification de l'échantillon comparable à celles obtenue avec des particules magnétiques.

### Conclusion générale :

Le procédé selon notre invention repose sur l'utilisation de la réactivité de la fonction diazo-méthyle vis-à-vis des acides (fonction carboxylique en particulier) en vue de capturer l'excès de marqueur après l'étape de marquage. Cette réactivité est connue de l'homme du métier. On peut citer, à titre d'exemple, le 4-(Diazomethyl)phenoxymethyl-polystyrène (Référence 17338, Fluka, Buchs, Suisse) utilisé pour lier de façon covalente les protéines par leurs liaisons carboxyliques. Cette résine a été utilisée par la Demanderesse dans le cadre d'expériences de capture d'acides nucléiques. L'idée d'utiliser la réactivité des fonctions diazo-méthyle vis-à-vis de support solide dans un processus de purification post-marquage est inconnu à ce jour.

La réaction est basée sur la conservation de la réactivité de la fonction diazo après l'étape de marquage. Cette conservation de la réactivité n'est pas du tout évidente, car une partie des fonctions pouvait être hydrolysée dans le milieu réactionnel durant l'étape de marquage initial. Le fait qu'une partie non négligeable des marqueurs n'ayant pas réagi avec les acides nucléiques soit encore réactive, et que l'immobilisation sur une phase solide s'effectue avec un rendement suffisant pour permettre une hybridation de l'échantillon, est un résultat surprenant.

La forte réactivité de la fonction diazo sur la fonction carboxylique permet de réaliser la réaction à température ambiante durant un temps limité à quelques minutes. Cette approche basée sur une liaison covalentesive est une innovation par rapport au techniques equivalents.

Les molécules de marqueurs étant séquestrées de façon covalente sur un support solide ou sur un polymère soluble, il n'est pas besoin de laver après purification. La suppression de l'étape de lavage est une innovation par rapport aux procédés de purification existants.

Le procédé, faisant l'objet de l'invention, étant basé sur une réaction chimique, il est plus spécifique et sélectif qu'une filtration, qu'une purification par exclusion de phase, ou qu'une adsorption sur support solide. Les risques de perte de l'échantillon biologique par adsorption sont diminués.

Enfin le support de purification peut être facilement intégré à un consommable, que ce soit un tube, dans le cas d'un procédé manuel, ou un composant de type carte, pour un protocole automatisé.

## Revendications

1. Procédé de marquage d'acides nucléiques d'intérêt contenues dans un échantillon biologique, consistant à :
a) disposer d'un récipient réactionnel,
b) immobiliser sur tout ou partie de la surface intérieure du récipient ou d'un support solide introduit dans ce récipient, des molécules de capture portant des fonctions anioniques et/ou acides, pouvant fixer un marqueur ou précurseur de marquage des acides nucléiques d'intérêt,
c) introduire dans ledit récipient réactionnel l'échantillon biologique mais également :
1) au moins un marqueur ou précurseur de marquage des acides nucléiques d'intérêt comprenant une fonction diazo, et
2) éventuellement tout ingrédient nécessaire au marquage ou pré-marquage des acides nucléiques d'intérêt,
d) incuber le contenu du récipient réactionnel,
e) immobiliser le marqueur ou précurseur de marquage n'ayant pas réagi avec les acides nucléiques d'intérêt par réaction de la fonction diazo avec les fonctions anioniques et/ou acides des molécules de capture pour former une liaison covalente, et
f) utiliser les acides nucléiques d'intérêt marquées, c'est-à-dire celles ayant réagi avec ledit marqueur ou précurseur de marquage, pour des étapes ultérieures.

2. Procédé de traitement d'un échantillon biologique contenant un mélange d'acides nucléiques d'intérêt et d'au moins un marqueur ou précurseur de marquage des acides nucléiques d'intérêt comprenant une fonction diazo, éventuellement associé à tout ingrédient nécessaire au marquage des acides nucléiques d'intérêt, consistant à :
a) disposer d'un récipient réactionnel,
b) immobiliser sur tout ou partie de la surface intérieure du récipient ou d'un support solide introduit dans ce récipient, des molécules de capture portant des fonctions anioniques et/ou acides pouvant fixer le marqueur ou précurseur de marquage des acides nucléiques d'intérêt,
c) introduire dans ledit récipient réactionnel l'échantillon biologique,
d) incuber le contenu du récipient réactionnel,
e) immobiliser le marqueur ou précurseur de marquage n'ayant pas réagi avec les acides nucléiques d'intérêt par fixation sur les molécules de capture, la fixation du marqueur ou précurseur de marquage n'ayant pas réagi avec les acides nucléiques d'intérêt, sur les molécules de capture s'effectuant par une liaison covalente, et
f) utiliser les acides nucléiques d'intérêt marquées, c'est-à-dire celles ayant réagi avec ledit marqueur ou précurseur de marquage, pour des étapes ultérieures.

3. Procédé selon l'une quelconque des revendications 1 à 2, **caractérisé en ce que**, préalablement à l'étape a), l'échantillon biologique est traité selon au moins l'une des étapes suivantes :
• le transfert depuis un autre récipient réactionnel amont,
• la lyse d'un matériel biologique complexe pour rendre les acides nucléiques d'intérêt accessibles et/ou détectables,
• la capture ou isolement des acides nucléiques d'intérêt, et/ou
• le traitement des acides nucléiques d'intérêt afin de rendre leur détection possible ou d'améliorer leur détection.

4. Procédé selon l'une quelconque des revendications 1 à 3, **caractérisé en ce que** l'étape f) est suivie d'au moins une étape ultérieure suivante :
• le transfert vers un autre récipient réactionnel aval,
• le marquage des acides nucléiques d'intérêt pré-marquées,
• la purification des acides nucléiques d'intérêt marquées ou pré-marquées, et/ou
• la détection des acides nucléiques d'intérêt marquées et hybridées sur des sondes de capture.

5. Procédé selon l'une quelconque des revendications 1 à 4, **caractérisé en ce que** les fonctions portées par la surface intérieure du récipient ou le support solide introduit dans ce récipient sont constituées par des fonctions carboxyliques et/ou sulfoniques.

6. Procédé selon l'une quelconque des revendications 1 à 5, **caractérisé en ce que** les acides nucléiques d'intérêt sont constituées par des acides nucléiques et/ou des fragments d'acides nucléiques.

7. Procédé selon la revendication 6, **caractérisé en ce que** les acides nucléiques et/ou les fragments d'acides nucléiques sont constitués par de l'ADN, de l'ARN, des polymères chimériques d'ADN-ARN.

8. Procédé selon la revendication 6, **caractérisé en ce que** les acides nucléiques et/ou les fragments d'acides nucléiques sont constitués par de l'ADN, de l'ARN, des polymères chimériques d'ADN-ARN contenant au moins un nucléotide thiophosphate, un LNA, un 2'-O-Me et/ou un dérivé méthylphosphonates.

9. Procédé selon l'une quelconque des revendications 6 à 8, **caractérisé en ce que**, préalablement à l'étape a), définie aux revendications 1 ou 2, l'échantillon biologique est traité selon au moins l'une des étapes suivantes :
• le transfert depuis un autre récipient réactionnel amont,
• la lyse du matériel biologique complexe, contenu par l'échantillon biologique, pour rendre les acides nucléiques accessibles,
• l'extraction des acides nucléiques à partir du matériel biologique complexe,
• l'amplification spécifique des acides nucléiques d'intérêt,
• la fragmentation desdits acides nucléiques d'intérêt ou amplicons, et/ou
• la transcription, ou la retrotranscription d'un acide nucléique d'intérêt, sans phénomène d'amplification notable.

10. Procédé selon l'une quelconque des revendications 6 à 9, **caractérisé en ce que** l'étape f), définie aux revendications 1 ou 2, est suivie d'au moins une étape ultérieure suivante :
• le transfert vers un autre récipient réactionnel aval,
• le marquage des acides nucléiques pré-marqués,
• la purification des acides nucléiques marqués ou pré-marqués,
• la détection des acides nucléiques marqués ou pré-marqués, hybridés sur des sondes de capture,
• la transcription ou la retrotranscription d'un acide nucléique d'intérêt, sans phénomène d'amplification notable, et/ou
• la détection en phase homogène des acides nucléiques marqués ou pré-marqués, avec ou sans utilisation de sondes de détection.

## Claims

1. Method for labelling nucleic acids of interest contained in a biological sample, consisting in:
a) providing a reaction vessel,
b) immobilizing, on all or part of the internal surface of the vessel or of a solid support introduced into this vessel, capture molecules bearing anionic and/or acid functions, which can bind a label or labelling precursor for the nucleic acids of interest,
c) introducing the biological sample into said reaction vessel, but also:
1) at least one label or labelling precursor for the nucleic acids of interest, comprising a diazo function, and
2) optionally, any ingredient required for labelling or prelabelling the nucleic acids of interest,
d) incubating the content of the reaction vessel,
e) immobilizing the label or labelling precursor which has not reacted with the nucleic acids of interest by reaction of the diazo function with the anionic and/or acid functions of the capture molecules so as to form a covalent bond, and
f) using the labelled nucleic acids of interest, i.e. those which have reacted with said label or labelling precursor, for subsequent steps.

2. Method for treating a biological sample containing a mixture of nucleic acids of interest and of at least one label or labelling precursor for the nucleic acids of interest, comprising a diazo function, optionally combined with any ingredient required for labelling the nucleic acids of interest, consisting in:
a) providing a reaction vessel,
b) immobilizing, on all or part of the internal surface of the vessel or of a solid support introduced into this vessel, capture molecules bearing anionic and/or acid functions, which can bind the label or labelling precursor for the nucleic acids of interest,
c) introducing the biological sample into said reaction vessel,
d) incubating the content of the reaction vessel,
e) immobilizing the label or labelling precursor which has not reacted with the nucleic acids of interest by binding to the capture molecules, the binding of the label or labelling precursor which has not reacted with the nucleic acids of interest, to the capture molecules taking place by means of a covalent bond, and
f) using the labelled nucleic acids of interest, i.e. those which have reacted with said label or labelling precursor, for subsequent steps.

3. Method according to either one of Claims 1 and 2, **characterized in that**, prior to step a), the biological sample is treated according to at least one of the following steps:
• transfer from another reaction vessel upstream,
• lysis of a complex biological material in order to make the nucleic acids of interest accessible and/or detectable,
• capture or isolation of the nucleic acids of interest, and/or
• treatment of the nucleic acids of interest in order to make their detection possible or to enhance their detection.

4. Method according to any one of Claims 1 to 3, **characterized in that** step f) is followed by at least one subsequent step below:
• transfer to another reaction vessel downstream,
• labelling of the prelabelled nucleic acids of interest,
• purification of the labelled or prelabelled nucleic acids of interest, and/or
• detection of the labelled nucleic acids of interest hybridized to capture probes.

5. Method according to any one of Claims 1 to 4, **characterized in that** the functions borne by the internal surface of the vessel or the solid support introduced into this vessel are constituted of carboxylic and/or sulphonic functions.

6. Method according to any one of Claims 1 to 5, **characterized in that** the nucleic acids of interest are constituted of nucleic acids and/or nucleic acid fragments.

7. Method according to Claim 6, **characterized in that** the nucleic acids and/or the nucleic acid fragments are constituted of DNA, RNA, DNA-RNA chimeric polymers.

8. Method according to Claim 6, **characterized in that** the nucleic acids and/or the nucleic acid fragments are constituted of DNA, RNA, DNA-RNA chimeric polymers containing at least one thiophosphate nucleotide, an LNA, a 2'-O-Me and/or a methylphosphonate derivative.

9. Method according to any one of Claims 6 to 8, **characterized in that**, prior to step a), defined in Claim 1 or 2, the biological sample is treated according to at least one of the following steps:
• transfer from another reaction vessel upstream,
• lysis of the complex biological material, contained by the biological sample, in order to make the nucleic acids accessible,
• extraction of the nucleic acids from the complex biological material,
• specific amplification of the nucleic acids of interest,
• fragmentation of said nucleic acids of interest or amplicons, and/or
• transcription or reverse transcription of a nucleic acid of interest, without any notable amplification phenomenon.

10. Method according to any one of Claims 6 to 9, **characterized in that** step f), defined in Claim 1 or 2, is followed by at least one subsequent step below:
• transfer to another reaction vessel downstream,
• labelling of the prelabelled nucleic acids,
• purification of the labelled or prelabelled nucleic acids,
• detection of the labelled or prelabelled nucleic acids hybridized to capture probes,
• transcription or reverse transcription of a nucleic acid of interest, without any notable amplification phenomenon, and/or
• homogeneous-phase detection of the labelled or prelabelled nucleic acids, with or without the use of detection probes.

## Patentansprüche

1. Verfahren zum Markieren von interessierenden Nukleinsäuren, die in einer biologischen Probe vorliegen, das Folgendes umfasst:
a) Verfügen über ein Reaktionsgefäß,
b) Immobilisieren von Fangmolekülen, die anionische und/oder saure Funktionen tragen und die einen Marker oder Vorläufer für die Markierung von interessierenden Nukleinsäuren fixieren können, auf der gesamten bzw. einem Teil der inneren Oberfläche des Gefäßes,
c) Einbringen der biologischen Probe, jedoch auch:
1) mindestens eines Markers oder einer Vorstufe für die Markierung von interessierenden Nukleinsäuren, der/die eine Diazofunktion umfasst, und
2) gegebenenfalls aller Bestandteile, die für die Markierung oder Vormarkierung der interessierenden Nukleinsäuren erforderlich sind,
in das Reaktionsgefäß,
d) Inkubieren des Inhalts des Reaktionsgefäßes,
e) Immobilisieren des Markers oder der Vorstufe für die Markierung, der/die nicht durch Reagieren der Diazofunktion mit den anionischen und/oder sauren Funktionen der Fangmoleküle unter Bildung einer kovalenten Bindung mit den interessierenden Nukleinsäuren reagiert hat, und
f) Einsetzen der markierten interessierenden Nukleinsäuren, also derjenigen, die mit dem Marker oder der Vorstufe für die Markierung reagiert haben, in nachfolgenden Schritten.

2. Verfahren zur Behandlung einer biologischen Probe, die eine Mischung von interessierenden Nukleinsäuren und mindestens einen Marker oder eine Vorstufe für die Markierung von interessierenden Nukleinsäuren, der/die eine Diazofunktion enthält, gegebenenfalls in Kombination mit allen Bestandteilen, die für die Markierung von interessierenden Nukleinsäuren erforderlich sind, enthält, das Folgendes umfasst:
a) Verfügen über ein Reaktionsgefäß,
b) Immobilisieren von Fangmolekülen, die anionische und/oder saure Funktionen tragen und die einen Marker oder Vorläufer für die Markierung von interessierenden Nukleinsäuren fixieren können, auf der gesamten bzw. einem Teil der inneren Oberfläche des Gefäßes,
c) Einbringen der biologischen Probe in das Reaktionsgefäß,
d) Inkubieren des Inhalts des Reaktionsgefäßes,
e) Immobilisieren des Markers oder der Vorstufe für die Markierung, der/die nicht mit den interessierenden Nukleinsäuren durch Fixieren auf den Fangmolekülen reagiert hat, wobei die Fixierung des Markers oder der Vorstufe für die Markierung, der/die nicht mit den interessierenden Nukleinsäuren reagiert hat, auf den Fangmolekülen durch eine kovalente Bindung erfolgt, und
f) Einsetzen der markierten interessierenden Nukleinsäuren, also derjenigen, die mit dem Marker oder der Vorstufe für die Markierung reagiert haben, in nachfolgenden Schritten.

3. Verfahren nach einem der Ansprüche 1 bis 2, **dadurch gekennzeichnet, dass** die biologische Probe vor Schritt a) gemäß mindestens einem der folgenden Schritte behandelt wird:
• Überführen von einem anderen, zuvor befindlichen Reaktionsgefäß,
• Lyse eines komplexen biologischen Materials, um die interessierenden Nukleinsäuren zugänglich und/oder nachweisbar zu machen,
• Fangen oder Isolieren der interessierenden Nukleinsäuren, und/oder
• Behandeln der interessierenden Nukleinsäuren, um ihren Nachweis zu ermöglichen oder ihren Nachweis zu verbessern.

4. Verfahren nach einem der Ansprüche 1 bis 3, **dadurch gekennzeichnet, dass** sich mindestens einer der folgenden Schritte an Schritt f) anschließt:
• Überführen in ein anderes, nachfolgend befindliches Reaktionsgefäß,
• Markieren der vormarkierten interessierenden Nukleinsäuren,
• Aufreinigung der markierten oder vormarkierten interessierenden Nukleinsäuren und/oder
• Nachweisen der markierten und auf Fangsonden hybridisierten interessierenden Nukleinsäuren.

5. Verfahren nach einem der Ansprüche 1 bis 4, **dadurch gekennzeichnet, dass** die von der inneren Oberfläche des Gefäßes getragenen Funktionen oder der in das Gefäß eingeführte feste Träger aus Carboxylfunktionen und/oder Sulfonfunktionen bestehen.

6. Verfahren nach einem der Ansprüche 1 bis 5, **dadurch gekennzeichnet, dass** die interessierenden Nukleinsäuren aus Nukleinsäuren und/oder Fragmenten von Nukleinsäuren bestehen.

7. Verfahren nach Anspruch 6, **dadurch gekennzeichnet, dass** die Nukleinsäuren und/oder die Fragmente von Nukleinsäuren aus DNA, RNA, chimären DNA-RNA-Polymeren bestehen.

8. Verfahren nach Anspruch 6, **dadurch gekennzeichnet, dass** die Nukleinsäuren und/oder die Fragmente von Nukleinsäuren aus DNA, RNA, chimären DNA-RNA-Polymeren, die mindestens ein Nukleotidthiophosphat, einer LNA, einem 2'-O-Me und/oder einem Methylphosphonatderivat bestehen.

9. Verfahren nach einem der Ansprüche 6 bis 8, **dadurch gekennzeichnet, dass** die biologische Probe vor Schritt a), der in den Ansprüchen 1 oder 2 definiert ist, gemäß mindestens einem der folgenden Schritte behandelt wird:
• Überführen von einem anderen, zuvor befindlichen Reaktionsgefäß,
• Lyse des komplexen biologischen Materials, das in der biologischen Probe enthalten ist, um die Nukleinsäuren zugänglich zu machen,
• Extrahieren der Nukleinsäuren aus dem komplexen biologischen Material,
• spezifisches Amplifizieren der interessierenden Nukleinsäuren,
• Fragmentieren dieser interessierenden Nukleinsäuren oder Amplikons, und/oder
• Transkribieren oder reverses Transkribieren einer interessierenden Nukleinsäure ohne nennenswerte Amplifikationserscheinung.

10. Verfahren nach einem der Ansprüche 6 bis 9, **dadurch gekennzeichnet, dass** sich an Schritt f), der in Anspruch 1 oder 2 definiert ist, mindestens ein anschließender Schritt wie folgt anschließt:
• Überführen in ein anderes, nachfolgend befindliches Reaktionsgefäß,
• Markieren der vormarkierten Nukleinsäuren,
• Aufreinigung der markierten oder vormarkierten Nukleinsäuren,
• Nachweisen der markierten oder vormarkierten und auf Fangsonden hybridisierten Nukleinsäuren,
• Transkribieren oder reverses Transkribieren einer interessierenden Nukleinsäure ohne nennenswerte Amplifikationserscheinung und/oder
• Nachweisen der markierten oder vormarkierten Nukleinsäuren in homogener Phase, und zwar mit oder ohne Einsatz von Nachweissonden.
